(19) Europäisches Patentamt
European Patent Office
Office européen des brevets

(11) **EP 4 589 348 A1**

(12) **EUROPEAN PATENT APPLICATION**

(43) Date of publication:
**23.07.2025 Bulletin 2025/30**

(21) Application number: **24181646.1**

(22) Date of filing: **12.06.2024**

(51) International Patent Classification (IPC):
**G02B 6/032** (2006.01)   **A61B 18/20** (2006.01)
**A61B 18/22** (2006.01)   **A61N 5/067** (2006.01)
**A61B 18/00** (2006.01)

(52) Cooperative Patent Classification (CPC):
**G02B 6/032; A61B 18/203; A61N 5/067;**
**G02B 6/262; G02B 6/4296;** A61B 2018/00011;
A61B 2018/00017; A61B 2018/00107;
A61B 2018/00458; A61B 2018/00476;
A61B 2018/00702; A61B 2018/00708;
A61B 2018/00714; A61B 2018/00726;
A61B 2018/00732;   (Cont.)

(84) Designated Contracting States:
**AL AT BE BG CH CY CZ DE DK EE ES FI FR GB
GR HR HU IE IS IT LI LT LU LV MC ME MK MT NL
NO PL PT RO RS SE SI SK SM TR**
Designated Extension States:
**BA**
Designated Validation States:
**GE KH MA MD TN**

(30) Priority: **18.01.2024 CN 202410076465**

(71) Applicant: **Beijing Laserconn Tech Co., Ltd.
Beijing 100192 (CN)**

(72) Inventors:
• **Yan, Li
Beijing (CN)**
• **KONG, Xiangli
Beijing (CN)**

(74) Representative: **Wang, Bo
Panovision IP
Ebersberger Straße 3
85570 Markt Schwaben (DE)**

(54) **SEMICONDUCTOR LASER COUPLING, TRANSMISSION, AND IMAGING APPARATUS USING LIQUID LIGHT GUIDE, AND DEVICE**

(57) The present disclosure discloses a semiconductor laser coupling, transmission, and imaging apparatus using a liquid light guide, and a device. The apparatus includes: a laser source; a liquid light guide, including a low refractive index tube body, two rod mirrors, light guide liquid, and an imaging lens; wherein the tube body has an accommodating cavity penetrating through the tube body in a lengthwise direction; the two rod mirrors are plugged at two ends of the accommodating cavity, and the light guide liquid fills the accommodating cavity; a first rod mirror located on the light entering side has a coupling end surface, and a second rod mirror located on the light exiting side has an exiting end surface. The present disclosure solves, by using the liquid light guide to replace an optical fiber, the problem that high-power semiconductor laser with poor beam quality cannot be coupled into the optical fiber.

FIG. 2B

(52) Cooperative Patent Classification (CPC): (Cont.)
    A61B 2018/00761; A61B 2018/206; G02B 6/0008;
    G02B 2006/0325

## Description

## BACKGROUND

## Technical Field

The present disclosure relates to a semiconductor laser coupling, transmission, and imaging apparatus using a liquid light guide, also relates to an electronic device using the same, and belongs to the field of semiconductor laser technologies.

## Related Art

Semiconductor laser devices typically adopt quartz optical fibers (referred to as optical fibers) for coupling and transmission. Beams emitted by one or more semiconductor laser chips are respectively collimated by a fast-axis collimator lens (FAC) and a slow-axis collimator lens (SAC) in two divergence angle directions, namely, a fast-axis direction and a slow-axis direction, to form a linear light spot, then are superimposed in the fast-axis direction after being stepwise reflected by multiple groups of reflectors, and are focused by a convex lens onto an end surface of an optical fiber, thus completing a coupling process of semiconductor laser.

Optical fibers used for semiconductor laser coupling are typically multimode optical fibers having a core diameter of 50 um to 1000 um. Due to the limitation of the rigidity and flexibility of a fiber core, it is very difficult for a fiber core diameter to exceed 1000 um. A numerical aperture (NA) of the optical fiber is usually 0.15, 0.22, and 0.37. A larger NA indicates that a larger laser incidence angle is acceptable. Coupling between the laser and the optical fiber needs to satisfy that a beam quality of the laser (which is usually measured in Beam Parameter Product (BPP)) is less than or equal to a beam quality that can be accepted by the optical fiber. A large-core optical fiber having an NA of 0.22 and a fiber core diameter of 1000 um is taken as an example, a full divergence angle of which is about 25 degrees. The BPP of laser that can be accepted by the optical fiber = 25 degrees* 1000 um = 25 mm * degree. Assuming that incoming laser is a linear light spot with a width of 10 mm and a full divergence angle of 3 degrees, the BPP of the incoming laser= 10 mm* 3 degrees = 30 mm* degree > 25 mm*degree. Therefore, the incoming laser cannot be completely coupled into the optical fiber, and a large coupling loss may be caused. If the divergence angle of the incoming laser is reduced to 1.5 degrees, the BPP of the incoming laser= 15 mm* degree < 25 mm* degree. Theoretically, the incoming laser can be completely coupled into the optical fiber end.

At present, medical laser commonly used in the dermatologic surgery, such as dye laser (585/595 nm), ruby laser (694 nm), alexandrite laser (755 nm), Nd: YAG laser (532 nm, 1064 nm), and conductor laser (within a band of 400 nm to 1500 nm, such as 635 nm, 810 nm, 940 nm, and 980 nm), often needs to be coupled, transmitted, and imaged through optical fibers, thus achieving uniform surface irradiation treatment. The semiconductor laser devices gradually become increasingly important sources of the medical laser due to their extremely high electro-optical conversion efficiency and abundant wavelength selections. However, as the application power of the semiconductor laser increases, it is rather difficult to meet the requirement for the beam quality of the semiconductor laser, and effective optical fiber coupling cannot be sufficiently achieved.

On the other hand, as an alternative to the optical fiber, a liquid light guide is widely used for ultraviolet curing lamps, excitation illumination for fluorescence detection, industrial cold light sources, surgical microscopes, Light-Emitting Diode (LED) illumination, and the like. The liquid light guide functions mainly as a gain medium and a waveguide for generation and transmission of low-power laser. A caliber of the liquid light guide is typically 2 mm, 3 mm, 5 mm, 8 mm, or the like, and the liquid light guide has a maximum full incidence angle that is up to 50 to 75 degrees. A liquid light guide having a caliber of 8 mm and a maximum full incidence angle of 75 degrees is taken as an example. This liquid light guide can accept a maximum incoming beam parameter product being up to 600 mm* degree, which is much greater than that of a single-mode large-core optical fiber. Thus, the liquid light guide can meet a coupling requirement of semiconductor laser with a larger BPP due to its larger incidence caliber and a larger NA. This provides a new solution for coupling and transmission of high-power semiconductor laser with poor beam quality.

## SUMMARY

The primary technical problem to be solved in the present disclosure is to provide a semiconductor laser coupling, transmission, and imaging apparatus using a liquid light guide.

Another technical problem to be solved in the present disclosure is to provide an electronic device using the semiconductor laser coupling, transmission, and imaging apparatus.

To achieve the foregoing technical objectives, the present disclosure adopts the following technical solutions:

According to a first aspect of embodiments of the present disclosure, a semiconductor laser coupling, transmission, and imaging apparatus using a liquid light guide is provided, including:

a laser source, configured to provide a shaped semiconductor laser beam;

a liquid light guide, including a low refractive index tube body, two rod mirrors, light guide liquid, and an imaging lens; wherein the tube body has an accommodating cavity penetrating through the tube body in a lengthwise direction; the two rod mirrors are plugged at two ends of the accommodating cavity, and the light guide liquid fills the accommodating cavity; and

an output portion, arranged on a light exiting side of the imaging lens to perform end surface imaging, wherein one of the two rod mirrors is located on a light entering side, and the other rod mirror is located on a light exiting side; a first rod mirror located on the light entering side has a coupling end surface, and a second rod mirror located on the light exiting side has an exiting end surface; the semiconductor laser beam is totally reflected inside the accommodating cavity and exits from the exiting end surface; and the imaging lens is located on a light exiting side of the exiting end surface for imaging.

Preferably, the laser source is a pulsed semiconductor laser device in a band of 400 to 1500 nm.

Preferably, the laser source has a beam parameter product of > 25 mm* degree, or in a range of 25 to 600 mm* degree.

Preferably, the laser source has average power of 200 W or less and peak power of 200 W to 5000 W.

Preferably, a focal point or beam waist of the semiconductor laser beam is located inside or outside the coupling end surface of the rod mirror.

Preferably, the rod mirror located on the light exiting side and the imaging lens satisfy the following conditions:

$$(1)\ OA > D + 2u*\tan(\theta),$$

$$(2)\ 1/u + 1/v = 1/f,$$

and

$$(3)\ D' = D*v/u,$$

wherein a clear aperture of the imaging lens is OA, and a focal length of the imaging lens is f; a diameter of the exiting end surface is D, and a half divergence angle is $\theta$; an imaging diameter is D'; a distance between the exiting end surface and a center of the imaging lens is u; and a focal length of an image side of the imaging lens is v.

Preferably, the light exiting side of the output portion is circular or rectangular to achieve uniform light exiting.

Preferably, the coupling end surface and the exiting end surface are each coated with an anti-reflective film to reduce an optical loss of an interface.

According to a second aspect of the present disclosure, an electronic device is provided, including the foregoing semiconductor laser coupling, transmission, and imaging apparatus, and a laser device control apparatus. The laser device control apparatus is configured to control the semiconductor laser coupling, transmission, and imaging apparatus.

Preferably, the electronic device is a laser medical device, and a peak power density of an imaging light spot of the laser medical device is greater than or equal to 100 W/cm2, and average power is less than or equal to 200 W.

Preferably, the electronic device further includes a distance sensor or a contact sensor, wherein the laser source is allowed to emit light only if a sensing signal of the distance sensor or the contact sensor satisfies a preset condition.

Compared with the prior art, the present disclosure has the following technical characteristics:

1. Using the liquid light guide to replace an optical fiber, the problem that high-power semiconductor laser with poor beam quality cannot be coupled into the optical fiber is solved.

2. Using the liquid light guide, irregular incoming light spots can be transmitted and homogenized into a flat-top output circular light spot.

3. A lens is used for end surface imaging, so that it is possible to achieve highly uniform power density distribution at an imaging position, thus meeting the requirement of the medical laser on the uniformity of light intensity distribution.

4. Using the pulsed laser, in conjunction with the design requirements of high peak power and low average power, an application need of the dermatologic surgery for the medical laser can be met.

## BRIEF DESCRIPTION OF THE DRAWINGS

FIG. 1 is a schematic diagram of an overall structure of a semiconductor laser coupling, transmission, and imaging apparatus according to a first embodiment of the present disclosure;
FIG. 2A is a schematic diagram of an overall structure of a liquid light guide in FIG. 1;
FIG. 2B is a longitudinal cross-sectional view of the liquid light guide in FIG. 2A and a schematic diagram of an imaging principle;
FIG. 3 is a flowchart of a semiconductor laser coupling, transmission, and imaging method according to a second embodiment of the present disclosure;
FIG. 4 is a schematic diagram of an overall structure of an electronic device according to a third embodiment of the present disclosure; and
FIG. 5 is a schematic structural diagram of a variation of the electronic device shown in FIG. 4.

## DETAILED DESCRIPTION

Technical contents of the present disclosure will be described in detail below with reference to the accompanying drawings and specific embodiments.

The technical ideas of the embodiments of the present disclosure are as follows: By full use of the characteristics of a liquid light guide, the liquid light guide is applied to coupling of high-power semiconductor laser with poor beam quality, and the semiconductor laser is further homogenized by transmission and is finally imaged with a lens at an output end, to form an extremely uniform laser spot. The embodiments of the present disclosure are particularly suitable for laser medical usage scenarios such as high-power laser body surface illumination of the dermatologic surgery.

First embodiment

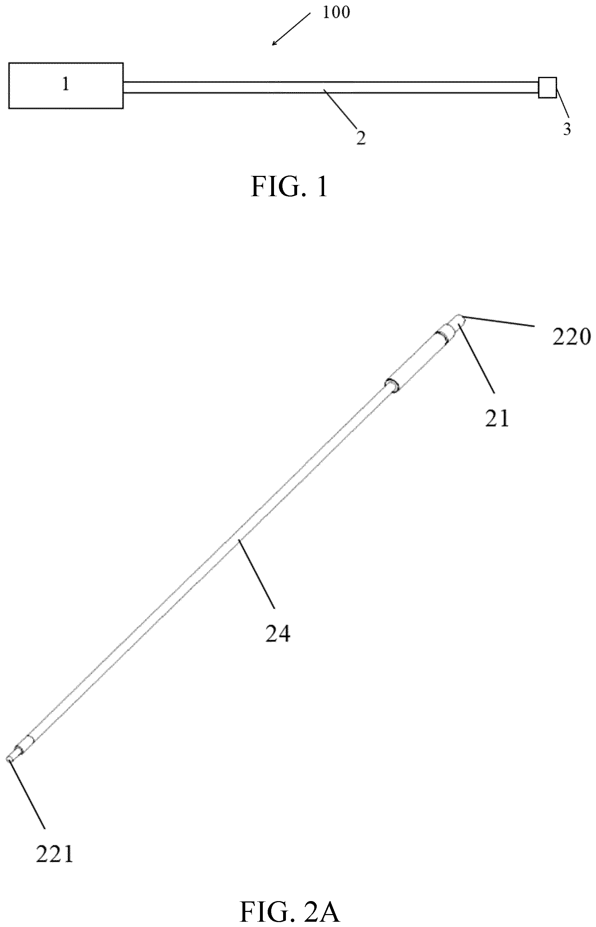

As shown in FIG. 1, the first embodiment of the present disclosure provides a semiconductor laser coupling, transmission, and imaging apparatus 100, which uses a liquid light guide to perform semiconductor laser coupling, transmission, and imaging. The apparatus includes a laser source 1, a liquid light guide 2, and an output portion 3.

The laser source 1 is configured to emit laser with a preset wavelength. The liquid light guide 2 is coupled to a light exiting side of the laser source 1 and is configured to randomly reflect incoming laser to form homogenized and decoherent outgoing laser. The output portion 3 is arranged at a light exiting side of the liquid light guide 2 and is configured to emit the laser to achieve end surface imaging, so that a highly uniform power distribution is achieved at an imaging position, particularly suitable for usage scenarios such as projection illumination, medical laser irradiation, criminal investigation devices, ultraviolet exposure, and optical etching. The laser emitted by the laser source 1 may have different optical and electrical parameters according to different requirements of the usage scenarios.

A usage scenario of laser treatment of the dermatologic surgery is taken as an example. Factors such as a selective photothermal effect and thermal relaxation time need to be considered during selection of working performance parameters of the laser source 1. The laser source 1 is controlled by a laser device control apparatus to output different laser beams. This will be described in detail later.

The selective photothermal effect refers to selecting an appropriate optical wavelength, energy, and pulse width according to the biological properties of different tissues, to ensure effective treatment of a diseased tissue and avoid an injury of surrounding normal tissues as much as possible. The thermal relaxation time of a human target tissue refers to a duration required by releasing half of a temperature rise after a target pigment is irradiated by laser. During treatment, a duration of a light pulse shall be less than the thermal relaxation time of a target structure, to prevent a non-selective injury of the surrounding normal tissues to the largest extent. For example, the thermal relaxation time of the epidermis is about 3 to 10 ms; the thermal relaxation time of the hair follicle is about 40 to 100 ms; and the thermal relaxation time of capillaries is about 0.1 ms to tens of millimeters according to different diameters. Thus, a laser pulse width is typically adjusted by the laser device control apparatus within a range of 0.1 ms to 100 ms, to use the selective photothermal effect of energy type laser to treat the target tissues.

In the usage scenario of treatment using the selective photothermal effect of the laser, such as laser hair removal, vasculopathy treatment, and epidermal pigment treatment, in order to avoid non-selective burns to human tissues (the skin, the blood vessels, or the like), it is typically required that the average power of the pulsed laser output by the device may not generally exceed 200 W. However, in order to ensure the treatment effect of the energy type laser, it is typically required that the peak power of the laser reaches 200 W to 5000 W. Furthermore, light spots that finally act on the human tissues are uniform, and the peak power density (namely, a power/light spot area ratio) on the light spots is typically $100 \ W/cm^2$ or more; and the pulse width is typically 0.1 ms to 100 ms, to significantly reduce non-selective heat injury to the surrounding tissues.

In this embodiment, the laser source 1 is high-power semiconductor laser that is appropriately focused after beam shaping and located in a range from visible light to infrared ray. Since a light-emitting spot of the single semiconductor laser device is shaped as a linear light spot with strong light in the middle and weak light at the edge. An overall light spot of a plurality of semiconductor laser devices that are superimposed is typically an alternately dark and bright striped rectangle or trapezoid. Therefore, a beam emitted by the laser source 1 needs to be properly focused before it is coupled into an input end of the liquid light guide. The focusing and coupling need to satisfy the following conditions:

(1) A focusing angle is less than a maximum incidence angle of the liquid light guide;
(2) the beam waist after focusing is located inside an end surface of the liquid light guide to reduce optical damage to the end surface; and
(3) A light spot of the beam at the coupling end surface of the liquid light guide needs to be smaller than a diameter of the end surface.

Specifically, in this embodiment, the beam of the laser source 1 has a preset beam parameter product which is a product of a diameter r of the beam waist of the laser and a full divergence angle $\alpha$. It can be under that an existing conventional coupling method for laser coupling is using a large-caliber optical fiber. However, the large-caliber optical fiber has a requirement for the beam quality (which is measured by the beam parameter product) of the laser. If the beam quality is low, it is impossible to use the large-caliber optical fiber to directly couple the laser device. The liquid light guide 2 used in this embodiment has a larger incidence caliber (typically 3 mm and above) than the optical fiber and a larger numerical aperture than the optical fiber, so that the liquid guide pipe is particularly suitable for coupling and transmitting laser with a BPP > 25 mm* degree. A liquid light guide with an incidence caliber of 3 to 8 mm and an incidence angle of 75 degrees is taken as an example, which can support incoming laser with a BPP < 600 mm* degree. This is far beyond the coupling range of the large-caliber optical fiber. In short, the beam parameter product of the laser source in this embodiment is in the range of 25 to 600 mm* degree, to couple laser that cannot be coupled by a large-core optical fiber and has poor beam quality.

It should be noted that, in other variations of the present disclosure, the above coupled focal beam waist may also be located outside the end surface of the liquid light guide, but not on the end surface. This can reduce the power density and avoid laser damage to the end surface and a film layer.

In this embodiment, the direct coupling for the liquid light guide 2 and the laser source 1 is particularly suitable for laser sources that cannot be coupled and transmitted through optical fibers because of the beam quality. In an embodiment of the present disclosure, the laser source 1 is a pulsed semiconductor laser device in a band of 400 to 1500 nm, which has the average power of 200 W or less and the peak power of 200 W to 5000 W, to avoid that extremely high average power causes heat injury to a patient or extremely high peak power causes denaturation or vacuolation of light guide liquid. This peak power does not destroy molecular bonds in a light guide solution (organic solution), thus avoiding the denaturation or vacuolation of the organic solution. Such a semiconductor laser source can meet the requirements of the energy type medical laser with the optical power density greater than 100 W/cm$^2$, in particular greater than or equal to 200 W/cm$^2$.

In this embodiment, the laser source 1 is applied to the following usage scenarios, including but not limited to:

1. Semiconductor laser for hair removal: A coupled light spot that is shaped by a semiconductor laser chip array (also referred to as bar) or a plurality of single core beams and is properly focused to have a focal point size of ≤ 3 mm enters the liquid light guide. The laser has a wavelength of 700 to 1100 nm, the peak power of 200 W to 5000 W, the pulse width of 1 to 100 ms, and the average power of 200 W or less. After being focused, a light spot on the skin surface has a diameter of 5 mm to 20 mm.

2. Long-pulse 980 nm or 1064 nm semiconductor laser for vasculopathy treatment and onychomycosis treatment: The peak power is 100 W to 2000 W; the average power is ≤ 50 W; the pulse width is 0.5 to 100 ms; and a focused light spot has a diameter of 2 to 5 mm.

3. Semiconductor laser for muscle and joint pain physiotherapy: Usually, the wavelength can be 635 to 650 nm and 810 nm; the peak power is ≤ 100 W; the average power is tens of watts for a large area of irradiation; and a light spot has a diameter of 10 mm to 200 mm, or the like.

It should be noted that, the power range of the laser source 1 is not limited to the examples in the use scenarios described above. In other use scenarios, the laser source 1 may also have the average power of 500 W or less, and the peak power may be 1 W to 50000 W.

The liquid light guide typically includes rod mirrors located at two end surfaces, a light guide solution, an optical plastic tube (typically of a fluoroplastic material, referred to herein as a "tube body") that covers the rod mirrors and the light guide solution, an end surface fastening joint, a protective sleeve or armor, and the like. For simplicity of description in this embodiment, only key components are described, and the remaining components may be added according to actual needs.

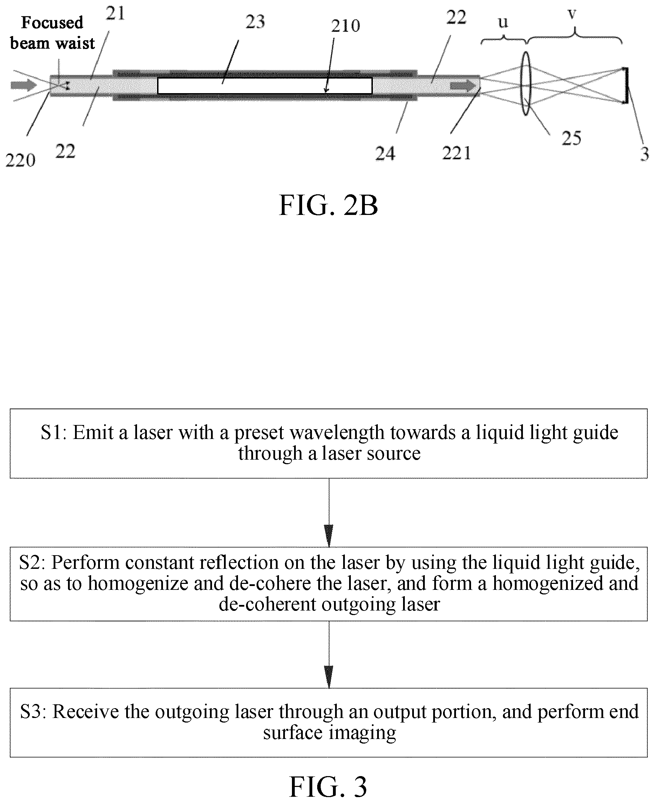

The liquid light guide 2 includes a tube body 21 made of low refractive index optical plastic (preferably low refractive index fluoroplastic), rod mirrors 22A or 22B located on two end surfaces, a light guide solution 23, a protective sleeve 24, and an imaging lens 25, as shown in FIG. 2A and FIG. 2B.

The refractive indexes of the rod mirrors and the refractive index of the light guide liquid are consistent or close to each other. Since the rod mirrors and the light guide liquid have a greater refractive index than the tube body, the beam can be totally reflected like in an optical fiber when transmitted inside the light guide liquid. A calculation formula for the interface reflectivity of the two media is: $R=(n1-n2)^2/(n1+n2)^2$. Where n1 and n2 are the true refractive indexes of the two media respectively. It is therefore necessary to ensure that the refractive indexes of the rod mirrors and the light guide liquid are consistent or are as close as possible, to reduce the reflection loss at an interface. Furthermore, the refractive index is greater than the refractive index of the optical plastic tube body to achieve internal total reflection in the light guide liquid.

In addition, the light guide liquid needs to use liquid that has a low absorption rate for the transmitted laser, and the liquid light guide has a limited length, typically within 3 m, to meet conventional medical use (a distance from the device to the patient). In this embodiment, a length of the tube body is about 1.5 to 3 m, which is the length desired in medical applications. The tube body 21 cannot be too short because the working principle of the liquid light guide is that the refractive index of the light guide solution 23 is less than the refractive index of the tube body 21, so that light is totally reflected for multiple times within the tube body to achieve uniform light exiting. If the length is insufficient, the uniform light exiting cannot be achieved.

An accommodating cavity 210 penetrating through the tube body is formed in the tube body 21 in a lengthwise direction. The two rod mirrors 22 are respectively plugged at two ends of the accommodating cavity 210. The light guide solution 23 fills the accommodating cavity 210 between the two rod mirrors 22.

In this embodiment, the liquid light guide 2 has a transmission efficiency greater than or equal to 85% to 90% (which is close to the efficiency of 85% to 95 of a single optical fiber)) for light emitted by the laser source 1, which is much better than the efficiency of 70% to 75% of an optical fiber bundle. Furthermore, the liquid light guide 2 has a lower incidence loss since it has no end surface holes for bundling an optical fiber bundle, and it is possible to avoid end surface burns on the holes of the optical fiber bundle during the transmission of high-power laser or high-energy pulsed laser.

In the above embodiments, specifically, the tube body 21 is made of transparent fluoroplastic having a low refractive index. The first rod mirror 22A on a light entering side and the second rod mirror 22B on a light exiting side are typically made of a quartz material. The sleeve 24 made of metal surrounds a periphery of the tube body 21. The first rod mirror 22A on the light entering side is coupled with the laser source 1, so that the laser is irradiated to the first rod mirror 22A and enters the light guide solution 23. The imaging lens 25 is arranged at one end of the second rod mirror 22B on the light exiting side. The light totally reflected by the light guide solution 23 enters the second rod mirror 22B on the light exiting side, and then exits to a target position such as the skin through the imaging lens 25. The transmissivities of the two rod mirrors are the same or approximately the same.

The first rod mirror 22A on the light entering side has a coupling end surface 220 (namely, an end surface facing the laser source 1). The second rod mirror 22B on the light exiting side has an exiting end surface 221 (namely, an end surface facing the imaging lens 25). The semiconductor laser beam is coupled to the coupling end surface 220, and a focal point or beam waist of the laser beam is located inside the coupling end surface 220 (namely, the focal point or beam waist of the laser beam is closer to the imaging lens 25 than the coupling surface 220), to reduce the laser power density of the end surface, thereby reducing optical damages to the end surface.

As shown in FIG. 2B, the light exiting from the second rod mirror 22B on the light exiting side (referring to the arrow direction in the figure) is converged to the skin surface through the imaging lens 25. The imaging lens 25 is located on a light exiting side of the second rod mirror 22B on the light exiting side, and the imaging lens and the second rod mirror are coaxial. The imaging lens 25 has a clear aperture that is large enough to receive all exiting beams for imaging on the end surface of the liquid light guide. Specifically, the imaging lens 25 satisfies the following three conditions:

$$(1)\ OA > D + 2u*\tan(\theta),$$

that is, imaging of all the beams

$$(2)\ 1/u + 1/v = 1/f,$$

that is, imaging formula

$$(3)\ D' = D*v/u,$$

that is, imaging magnification relationship where a clear aperture of the imaging lens 25 is OA, and a focal length of the imaging lens is f; a diameter of the exiting end surface 221 is D, and a half divergence angle is $\theta$; an imaging diameter is D'; a distance (a focal length of an object side of the imaging lens) between the exiting end surface 221 and a center of the imaging lens is u; and a focal length of an image side of the imaging lens is v.

With the above design, it is possible to effectively complete reliable coupling between the semiconductor laser to the liquid light guide and achieve transmission loss as little as possible. During the transmission of the semiconductor laser, irregular inner wall total reflection is performed using a side wall of an optical plastic sleeve that slightly deforms to complete light spot homogenization, finally forming uniform light intensity distribution on a cross section and outputting light from the output portion. At the output portion, uniform round image points are formed using the imaging lens, so that uniform optical irradiation can be achieved.

Moreover, in this embodiment, an imaging light spot is mainly used for laser medical treatment. The peak power density of the imaging light spot is greater than or equal to 100 W/cm2 (to achieve effective treatment) and the average power is less than or equal to 200 W (to avoid burning the human body), to achieve effective and safe irradiation to the human skin. More preferably, certain skin cooling measures are taken, such as cold air, sapphire contact cooling, intermittent ice compress, or spraying of a skin refrigerant.

More preferably, the output portion 3 located on the light exiting side of the liquid light guide 2 has a circular or rectangular outline of a light exiting surface. In this way, the light exiting of the output portion 3 is more uniform to avoid local burns.

It should be noted that, in the above embodiment, a preset length of the tube body 21 is positively related to the degree of homogenization of the laser, and a preset inner diameter is negatively related to the degree of homogenization of the laser. It can be understood that a longer tube body 21 has a smaller inner diameter, and the number of reflections of the laser in the accommodating cavity is larger, so that the homogenization performed on the laser is better, and vice versa, the homogenization performed on the laser is poorer. For a particular use, a tube body 21 with suitable length and internal diameter is selected according to a need.

In the above embodiments, preferably, the coupling end surface 220 and the exiting end surface 221 are each coated with an anti-reflective film (which is selected according to the wavelength of the laser), so that the reflection loss of an air interface is reduced when the laser beam is coupled to the first rod mirror 22A and exits from the second rod mirror 22B.

On the other hand, in other variations of the present disclosure, the application of the output portion is not necessarily limited to the position of the circular image point, but may be at an inner side or an outer side, to achieve light spots with different sizes. A light spot on the inner side has a clear boundary, but there is a medical risk caused by a high-power density or energy density at a distant focal point. A light spot on the outer side has a blurred boundary, but is safer for mis-operations of laser treatment.

It can be understood that the semiconductor laser coupling, transmission, and imaging apparatus provided by the embodiments of the present disclosure can be used in a variety of scenarios. For example, the semiconductor laser coupling, transmission, and imaging apparatus can be applied to medical and beauty scenarios, such as laser hair removal, vasculopathy treatment, and pigment treatment by high-power, high-energy-density, uniformly distributed light spots.

Second embodiment

As shown in FIG. 3, based on the above first embodiment, the second embodiment of the present disclosure provides a semiconductor laser coupling, transmission, and imaging method, specifically including step S1 to step S3:

S1: Emit a laser with a preset wavelength towards the liquid light guide 2 through the laser source 1.
S2: Perform inner wall total reflection on the laser for multiple times by using an inner surface of the liquid light guide 2 with a slightly deformed inner wall, to homogenize the laser, and form a homogenized outgoing laser at the exiting end of the liquid light guide 2.
S3: Receive the outgoing laser through the output portion 3, and perform end surface imaging.

Third embodiment

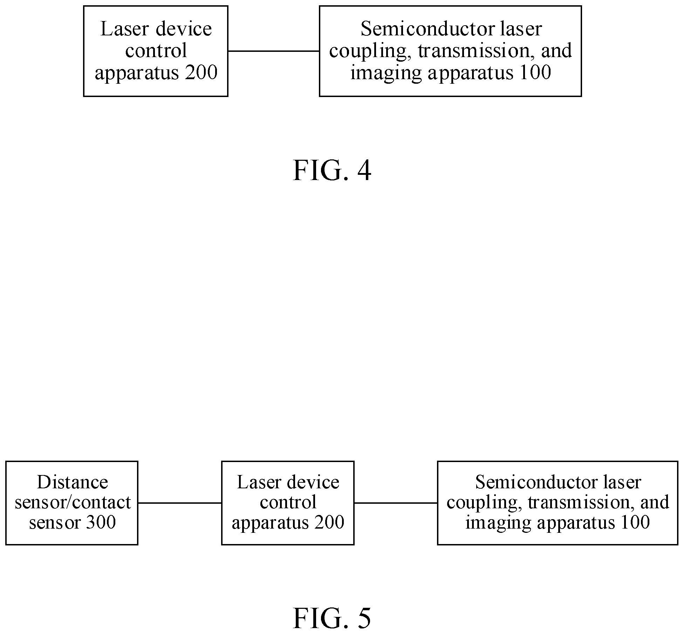

Based on the above first embodiment, the third embodiment of the present disclosure further provides an electronic device. As shown in FIG. 4, the electronic device includes the above semiconductor laser coupling, transmission, and imaging apparatus 100 and a laser device control apparatus 200. The laser device control apparatus 200 is configured to control the semiconductor laser coupling, transmission, and imaging apparatus 100.

The control of laser power parameters is mainly based on the principle of the selective photothermal effect, so that output parameters of the final laser need to be strictly controlled. The laser device control apparatus 200 is mainly responsible for constant current driving and adjustment of a semiconductor laser device, including starting and stopping the laser and controlling parameters such as the peak power, the pulse width, the pulse frequency, and the duty cycle, and including cooling and temperature control of the laser source 1 to ensure stable laser power outputting. The semiconductor laser coupling, transmission, and imaging apparatus 100 is responsible for coupling, transmission (homogenization), and output end surface imaging of the semiconductor laser. According to different requirements of applications, by changing the parameters of the laser device control apparatus 200, the electronic device is set to have different electrical and optical properties.

Specifically, the laser device control apparatus 200 controls start or stop of the laser source 1 in the semiconductor laser coupling, transmission, and imaging apparatus 100, and is also configured to control the optical and electrical parameters such as the peak power, the pulse width, the frequency, and the duty ratio of the laser source 1 to meet the requirements of technical indicators of different application scenarios.

In different embodiments of the present disclosure, the electronic device may be a medical laser therapeutic instrument such as a laser skin therapeutic instrument and a vascular therapeutic instrument. As shown in FIG. 5, in a variation of the electronic device described above, a distance sensor or contact sensor 300 connected to the laser device control apparatus 200 may be provided to establish an interlock protection mechanism. The interlock protection mechanism here means that: The laser source 1 is allowed to emit light only if a sensing signal of the distance sensor or the contact sensor satisfies a preset condition (for example, a treated area is within a preset working distance range), to ensure that a laser spot is consistent with the preset condition.

In summary, the semiconductor laser coupling, transmission, and imaging apparatus using the liquid light guide, and the device provided by the embodiments of the present disclosure have the following beneficial effects:

1. Using the liquid light guide to replace an optical fiber, the problem that high-power semiconductor laser with poor beam quality cannot be coupled into the optical fiber is solved.

Specifically, compared with a single optical fiber, the liquid light guide has a larger caliber of incidence. Therefore, the liquid light guide has a low requirement for the beam quality of incoming light, low coupling difficulty, and high coupling efficiency, and can bear higher peak incident power.

Compared with an optical fiber or an optical fiber bundle, the irregular internal wall reflection of the liquid light guide can eliminate the coherence of the laser, thus facilitating beam homogenization and de-coherence. The full-caliber incidence avoids filling ratio loss of the optical fiber bundle.

2. Using the liquid light guide, irregular incoming light spots can be transmitted and homogenized into a flat-top output circular light spot, which achieves better power homogenization and flat-top outputting.
3. A lens is used for end surface imaging, so that it is possible to achieve highly uniform power density distribution at an imaging position, thus meeting the requirement of the medical laser on the uniformity of light intensity distribution.
4. Using the pulsed laser, in conjunction with the design requirements of relatively low peak power (avoiding the denaturation of the organic solution) and relatively high average power (avoiding a failure of meeting the requirement of the application scenario), an application need of the dermatologic surgery for the medical laser can be met.

The semiconductor laser coupling, transmission, and imaging apparatus using the liquid light guide, and the device provided by the present disclosure are described in detail above. For those of ordinary skill in the art, any obvious changes made to the present disclosure without deviating from its substantive content will constitute infringement of the patent rights of the present disclosure, and they will bear corresponding legal responsibilities.

## Claims

1. A semiconductor laser coupling, transmission, and imaging apparatus using a liquid light guide, comprising:

   a laser source, configured to provide a shaped semiconductor laser beam;
   a liquid light guide, comprising a low refractive index tube body, two rod mirrors, light guide liquid, and an imaging lens; wherein the tube body has an accommodating cavity penetrating through the tube body in a lengthwise direction; the two rod mirrors are plugged at two ends of the accommodating cavity, and the light guide liquid fills the accommodating cavity; and
   an output portion, arranged on a light exiting side of the imaging lens to perform end surface imaging,
   wherein one of the two rod mirrors is located on a light entering side, and the other rod mirror is located on a light exiting side; a first rod mirror located on the light entering side has a coupling end surface, and a second rod mirror located on the light exiting side has an exiting end surface; the semiconductor laser beam is totally reflected inside the accommodating cavity and exits from the exiting end surface; and
   the imaging lens is located on a light exiting side of the exiting end surface for imaging.

2. The semiconductor laser coupling, transmission, and imaging apparatus according to claim 1, wherein the laser source is a pulsed semiconductor laser device in a band of 400 to 1500 nm.

3. The semiconductor laser coupling, transmission, and imaging apparatus according to claim 2, wherein the laser source has a beam parameter product of > 25 mm* degree, or in a range of 25 to 600 mm* degree.

4. The semiconductor laser coupling, transmission, and imaging apparatus according to claim 3, wherein the laser source has average power of 200 W or less and peak power of 200 W to 5000 W.

5. The semiconductor laser coupling, transmission, and imaging apparatus according to claim 3, wherein the laser source has average power of 500 W or less and peak power of 1 W to 50000 W.

6. The semiconductor laser coupling, transmission, and imaging apparatus according to claim 3, wherein a focal point or beam waist of the semiconductor laser beam is located inside or outside the coupling end surface of the rod mirror.

7. The semiconductor laser coupling, transmission, and imaging apparatus according to claim 1, wherein the rod mirror located on the light exiting side and the imaging lens satisfy the following conditions:

$$(1)\ OA > D + 2u^*\tan(\theta),$$

$$(2)\ 1/u + 1/v = 1/f,$$

and

$$(3)\ D' = D^*v/u,$$

wherein a clear aperture of the imaging lens is OA, and a focal length of the imaging lens is f; a diameter of the exiting end surface is D, and a half divergence angle is θ; an imaging diameter is D'; a distance between the exiting end surface and a center of the imaging lens is u; and a focal length of an image side of the imaging lens is v.

8. The semiconductor laser coupling, transmission, and imaging apparatus according to claim 7, wherein the light exiting side of the output portion is circular or rectangular to achieve uniform light exiting.

9. The semiconductor laser coupling, transmission, and imaging apparatus according to claim 7, wherein the coupling end surface and the exiting end surface are each coated with an anti-reflective film to reduce an optical loss of an interface.

10. An electronic device, comprising the semiconductor laser coupling, transmission, and imaging apparatus according to claim 1, and a laser device control apparatus, wherein the laser device control apparatus is configured to control the semiconductor laser coupling, transmission, and imaging apparatus.

11. The electronic device according to claim 10, wherein the electronic device is a laser medical device, and a peak power density of an imaging light spot of the laser medical device is greater than or equal to 100 W/cm2, and average power is less than or equal to 200 W.

12. The electronic device according to claim 10, further comprising a distance sensor or a contact sensor, wherein the laser source is allowed to emit light only if a sensing signal of the distance sensor or the contact sensor satisfies a preset condition.

FIG. 1

FIG. 2A

Focused beam waist 21 23 210 22 u v

220 22 24 221 25 3

## FIG. 2B

| S1: Emit a laser with a preset wavelength towards a liquid light guide through a laser source |
| --- |

| S2: Perform constant reflection on the laser by using the liquid light guide, so as to homogenize and de-cohere the laser, and form a homogenized and de-coherent outgoing laser |
| --- |

| S3: Receive the outgoing laser through an output portion, and perform end surface imaging |
| --- |

## FIG. 3

| Laser device control apparatus 200 | | Semiconductor laser coupling, transmission, and imaging apparatus 100 |
|---|---|---|

FIG. 4

| Distance sensor/contact sensor 300 | | Laser device control apparatus 200 | | Semiconductor laser coupling, transmission, and imaging apparatus 100 |
|---|---|---|---|---|

FIG. 5

**EUROPEAN SEARCH REPORT**

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | DE 22 57 805 A1 (COMMW SCIENT IND RES ORG) 7 June 1973 (1973-06-07)<br>* figures 1, 3, 5 *<br>* p. 10, 3rd par. *<br>* p. 14, 2nd par. * | 1-10,12 | INV.<br>G02B6/032<br>A61B18/20<br>A61B18/22<br>A61N5/067<br>A61B18/00 |
| X | EP 3 232 239 A1 (LYOCON S R L [IT]) 18 October 2017 (2017-10-18)<br>* figures 1, 2a, 2b *<br>* paragraph [0004] *<br>* paragraph [0001] *<br>* paragraphs [0010] - [0011] *<br>* paragraphs [0018] - [0019] *<br>* paragraph [0042] *<br>* paragraph [0053] *<br>* paragraph [0058] * | 1-12 | |
| A | US 2012/143004 A1 (GUPTA AMITAVA [US] ET AL) 7 June 2012 (2012-06-07)<br>* paragraph [0056] * | 1-12 | |
| A | CN 103 398 314 A (SHANGHAI HAICHAO NEW TECHNOLOGY RES INST) 20 November 2013 (2013-11-20)<br>* figure 1 * | 1-12 | **TECHNICAL FIELDS SEARCHED (IPC)**<br><br>G02B<br>A61B<br>A61N<br>F21V |
| A | CN 103 440 895 A (UNIV HARBIN SCIENCE & TECH) 11 December 2013 (2013-12-11)<br>* figure 1 * | 1-12 | |
| A | JP H05 313015 A (BRIDGESTONE CORP) 26 November 1993 (1993-11-26)<br>* figures 1-3 * | 1-12 | |

The present search report has been drawn up for all claims

| Place of search | Date of completion of the search | Examiner |
|---|---|---|
| The Hague | 7 November 2024 | Dregely, Daniel |

CATEGORY OF CITED DOCUMENTS

X : particularly relevant if taken alone
Y : particularly relevant if combined with another
document of the same category
A : technological background
O : non-written disclosure
P : intermediate document

T : theory or principle underlying the invention
E : earlier patent document, but published on, or
after the filing date
D : document cited in the application
L : document cited for other reasons

& : member of the same patent family, corresponding
document

EPO FORM 1503 03.82 (P04C01)

**ANNEX TO THE EUROPEAN SEARCH REPORT
ON EUROPEAN PATENT APPLICATION NO.**                    EP 24 18 1646

This annex lists the patent family members relating to the patent documents cited in  the above-mentioned European search report.
The members are as contained in the European Patent Office EDP file on
The European Patent Office is in no way liable for these particulars which are merely given for the purpose of information.

07-11-2024

| Patent document cited in search report | | Publication date | Patent family member(s) | | Publication date |
|---|---|---|---|---|---|
| DE 2257805 | A1 | 07-06-1973 | DE | 2257805 A1 | 07-06-1973 |
| | | | JP | S4981045 A | 05-08-1974 |
| | | | NL | 7216191 A | 01-06-1973 |
| EP 3232239 | A1 | 18-10-2017 | EP | 3232239 A1 | 18-10-2017 |
| | | | ES | 2927853 T3 | 11-11-2022 |
| | | | HK | 1245411 A1 | 24-08-2018 |
| US 2012143004 | A1 | 07-06-2012 | AR | 084502 A1 | 22-05-2013 |
| | | | AU | 2011336505 A1 | 20-06-2013 |
| | | | BR | 112013013584 A2 | 06-09-2016 |
| | | | CA | 2819514 A1 | 07-06-2012 |
| | | | CN | 103384491 A | 06-11-2013 |
| | | | EP | 2645919 A1 | 09-10-2013 |
| | | | JP | 6031445 B2 | 24-11-2016 |
| | | | JP | 2013545558 A | 26-12-2013 |
| | | | KR | 20140001963 A | 07-01-2014 |
| | | | MX | 338575 B | 22-04-2016 |
| | | | MX | 343835 B | 24-11-2016 |
| | | | PT | 2645919 T | 12-03-2020 |
| | | | RU | 2013126208 A | 10-01-2015 |
| | | | SG | 190420 A1 | 31-07-2013 |
| | | | SG | 10201509874Y A | 28-01-2016 |
| | | | US | 2012143004 A1 | 07-06-2012 |
| | | | WO | 2012075280 A1 | 07-06-2012 |
| | | | ZA | 201303880 B | 30-07-2014 |
| CN 103398314 | A | 20-11-2013 | NONE | | |
| CN 103440895 | A | 11-12-2013 | NONE | | |
| JP H05313015 | A | 26-11-1993 | NONE | | |

EPO FORM P0459

For more details about this annex : see Official Journal of the European Patent Office, No. 12/82